# EUROPEAN PATENT APPLICATION

(11) **EP 4 445 923 A1**
(43) Date of publication of application: **16.10.2024**
(21) Application number: 23168135.4
(22) Date of filing: 14.04.2023
(51) Int. Cl.: A61L 27/46, A61L 27/56, B33Y 70/00

(54) **DIMENSIONALLY STABLE GELATIN-BASED BONE IMPLANT**

(71) Applicant: Gottfried Wilhelm Leibniz Universität Hannover, 30167 Hannover (DE)
(72) Inventor: Naolou, Toufik, 30419 Hannover (DE); Lee-Thedieck, Cornelia, 31515 Wunstorf (DE); Schadzek, Nadine, 30539 Hannover (DE); Pepelanova, Iliyana, 31582 Nienburg (DE); Frommer, Miriam, 30161 Hannover (DE); Lötz, Franziska, 30419 Hannover (DE)
(74) Representative: Taruttis, Stefan Georg

(57) **Abstract**

The invention relates to a Gelatin-based composition suitable for use in the treatment of a bone defect site. The composition contains hydroxyapatite particles, preferably hydroxyapatite nanoparticles and has the advantage of being pourable, e.g. injectable and/or extrudable and/or printable, e.g. for use in a 3D-printing process or injection-moulding process, at elevated temperatures, e.g. at 37 to 42 °C, and to be dimensionally stable upon cooling, e.g. to 5 °C or below to 0 °C or 1 °C, wherein the solidification upon cooling is sufficiently fast for generating dimensionally stable structures, e.g. upon cooling by depositing the pourable composition on a cooling carrier or platform.

## Description

The present invention relates to a Gelatin-based composition suitable for use in the treatment of a bone defect site, e.g. for use as a bone implant and preferably to a process for forming dimensionally stable structures comprising or consisting of the Gelatin-based composition, e.g. by 3D-printing or by moulding in a mould, which is e.g. an open or closed mould.

The Gelatin-based composition contains hydroxyapatite particles, preferably hydroxyapatite nanoparticles and has the advantage of being pourable, e.g. injectable and/or extrudable and/or printable, e.g. for use in a 3D-printing process or injection-moulding process, at elevated temperatures, e.g. at 37 to 42 °C, and to be dimensionally stable upon cooling, e.g. to 5 °C or below to 0 °C or 1 °C, wherein the solidification upon cooling is sufficiently fast for generating dimensionally stable structures, e.g. upon cooling by depositing the pourable composition on a cooling carrier or platform, e.g. a platform having a temperature of 5 °C to 0 °C or to 1 °C, e.g. wherein the platform is e.g. temperature-controlled by being equipped with a temperature-control device, e.g. an internal chamber for a thermostatically controlled circulating fluid.

Optionally, the platform temperature is controlled using a temperature-controlled chamber, e.g., having an interior temperature of 5 °C to 0 °C or to 1 °C.

Furthermore, the composition of the invention has the advantage of generating structures which are dimensionally stable also at 37 °C, e.g. due to radical cross-linking, e.g. induced by UV irradiation.

### State of the art

Wüst et al., Acta Biomaterialia 10 (2014) 630-640 describe a mixture of 10% w/v type A gelatin with 4 or 8% w/v hydroxyapatite and 2% w/v alginate dissolved in PBS and expelling the mixture at 40°C through a syringe and after deposition cooling to 10°C, with subsequent immersion in a Ca²⁺ solution for solidification of the alginate. The results show that after printing, the mixture is not sufficiently stable to maintain the filamentous structure with high fidelity.

Hoorick et al., Acta Biomater 97, 46-73 (2019) review cross-linkable gelatin derivatives.

### Object of the invention

The invention has the object of providing a composition that can be formed into a threedimensionally stable structure, preferably containing interconnected macropores, e.g. through-holes, and its composition is suitable for growth of adherent osteogenic cells. Preferably, the composition shall be suitable for generating a three-dimensional structure by extrusion and deposition, preferably by 3D-printing, or by moulding.

### Description of the invention

The invention achieves the object by the features of the claims and specifically by providing a composition which in its uncured state comprises or consists of 5% to 30% cross-linkable gelatin derivatives, especially gelatin methacrylate (GelMA) and/or gelatin acrylate , 1% to 10% nanoparticular hydroxy apatite (nHA), 1% to 5% Carbomer), and preferably a radical polymerization initiator, e.g. at 0.8 to 1.2 %. By radical cross-linking, the composition is suitable for producing, e.g. by radical cross-linking, an elastic scaffold that promotes bone formation in contact with cells, e.g. after implantation into a human, especially into a bone defect site, promotes bone formation at the site of implantation, i.e. promotes bone formation on and in the structure of the composition in its cured state. Accordingly, the composition in its cured state is suitable for use as a bone implant, e.g. for filling defect sites in a bone. Due to the elasticity of the composition in its cured state, it is suitable for use as a bone implant, the use comprising a step of bending or folding the bone implant and, subsequent to depositing the bended or folded implant at a site of implantation, comprising the step of relaxation into the original three-dimensional shape of the implant, e.g. until limited by the site of implantation. Herein, the cured state is preferably generated by radical cross-linking, also referred to as radically cured state. Optionally, e.g. for use in the treatment of a bone defect or cartilage defect, the composition of the invention can be for use in the treatment, wherein the treatment comprises the step of filling a defect site in bone or in cartilage with the composition in its uncured state and subsequently initiating radical cross-linking, resulting in the production of an implant comprising or consisting of the composition in its cured state at the site of the bone or cartilage defect.

Generally, it is preferable that the pH of the composition is pH 7, e.g. by adjusting the pH prior to depositing the composition on a carrier.

The cross-linkable gelatin can e.g. be selected from cross-linkable gelatin derivatives as described in Hoorick et al (loc cit), e.g. (meth)acryl-amidated gelatin, thiolated and aminated gelatin, PEG-acrylate-coupled gelatin, thiobutyrolacton-coupled gelatin, preferably acrylated gelatin derivatives, which is e.g. gelatin acrylate, gelatin methacrylate or a mixture of least two of these.

The composition in its uncured state, especially for use in expelling through a nozzle, e.g. by extrusion and/or 3D-printing, preferably has a viscosity of 20 000 Pas at a shear rate of 0/s up to 1 Pas at a shear rate of 2000/s at 37°C. Preferably, the viscosity is measured between a pair of spaced-apart parallel plates, one plate of which is driven to rotate at the shear rate, wherein the plates which are e.g. arranged with a gap of 0.5 mm.

The composition in its cured state, preferably obtained by cooling immediately after extrusion, e.g. by deposition on a cooled carrier platform, and with curing obtained by radical polymerization, has a shear modulus of 5 to 500 kPa, a storage modulus G' of 10 to 300 kPa, e.g. measured at 15 min subsequent to initiation of the radical polymerization, and a loss modulus G" of 0.1 to 3 kPa, each measured at 37°C by small amplitude oscillatory shear measurement. The curing by radical polymerization can be initiated directly after the uncured composition exits a nozzle, after deposition of the composition on a carrier, herein also referred to as and represented by a platform, and/or into a mould. Generally, the composition shows self-healing properties in the uncured state, especially as determined by stressrelaxation experiments with alternate cycles of stress (200%), relaxation (1%) amplitude. Preferably, the composition in its cured state has a storage modulus G' of 10 to 300 kPa and a loss modulus G" of 0.1 to 3 kPa, also after at least 5 deformation cycles of 200% deformation in relation to the relaxed shape and back to a non-deformed shape, each deformation cycle executed with a continuous movement over 60 s, and, subsequent to the deformation cycles, executing over 120 s a low recovery strain of 1% deformation in relation to the relaxed shape. Therein, a deformation of 200% in relation to the relaxed shape of the composition in its cured state is a deformation about one axis, e.g. bending about one axis, for a distance equal to double the thickness of the relaxed shape, and a strain for 1% deformation is a strain sufficient for bending about one axis for a distance equal to 1% of the thickness of the relaxed shape.

Preferably, the viscosity, the storage modulus and the loss modulus of the composition in its cured state are determined by small amplitude shear measurement, e.g. using a modular MCR 302 rheometer (Anton Paar, Austria), with parallel plate configuration, spaced by 0.5 mm, wherein the composition fills the spacing as a bubble-free and cured layer. A water trap was used to cover the sample gap in order to prevent evaporation. Preferably, the uncured composition is extruded, e.g. using a syringe, onto a lower plate to form a bubble-free layer, followed by lowering of the upper plate into the spacing of 0.5 mm, then irradiating the composition for initializing radical cross-linking, allowing the cross-linking to proceed to completion, and applying strain to one of the plates. It was found that cross-linking of a composition containing a radical polymerization initiator is essentially complete after 15 min at 37°C. The strain applied is sufficient for e.g. a range of 1 % to 1000 % deformation of the composition arranged in the spacing between the parallel plates by movement of one of the plates in parallel to the other plate at one frequency of movement or over a range of frequencies, wherein the frequency is in the range of 1 Hz to 10 Hz, preferably performed as a frequency sweep over the range of 1 to 10 Hz with 1% deformation, or performed as amplitude sweeps at 1 Hz and variable deformation from 0.1% to 1000%. Generally, measurements are performed at 37°C.

In an embodiment, the physical crosslinking process is initiated by cooling directly after the uncured composition exits a nozzle and during its deposition on a platform, and initiating the radical polymerization, e.g. by irradiating the uncured composition subsequent to its deposition, e.g. subsequent to cooling to below 20 °C, below 15 °C or below 10 °C or below 5 °C, wherein the cooling can be by deposition on the cooling platform.

The components of the composition can be varied in order to adjust the composition in the uncured state to have a viscosity suitable for expelling it through a nozzle, e.g. a nozzle of a 3D-printer for depositing the flow of the composition exiting the nozzle on a carrier. The viscosity of 20 000 Pas at a shear rate of 0/s up to 1 Pas at a shear rate of 2000/s at 37°C is suitable for the process because it is sufficiently flowable for expelling through a nozzle, e.g. sufficiently flowable for extrusion, and is sufficiently viscous, also described as sufficiently thick, to maintain its shape while passing the distance to a carrier and for maintaining its shape after deposition on the carrier, which shape can be fixated by cross-linking. The viscosity of the composition is adapted to generate a stable stream of a stable shape, e.g. a cylindrical cross-section filament, of the composition when expelled through a nozzle, e.g. extruded, which composition can be deposited on a carrier while essentially maintaining its shape.

The viscosity of the composition in its uncured state and, subsequent to initiating radical polymerization, its viscosity in the cured state, can be adjusted by the contents of gelatin methacrylate, as a higher content of gelatin methacrylate results in an increase of the viscosity of the composition in its uncured state, by the contents of nano hydroxy apatite, as a higher content of nano hydroxy apatite results in a decrease of the viscosity of the composition in its uncured state, and by the contents of carbomer, as a higher content of carbomer results in an increase of the viscosity of the composition in its uncured state and e.g. compensates for a decrease of viscosity caused by a higher content of nano hydroxy apatite.

Herein, unless indicated otherwise, percentages (%) are weight per weight (w/w), remainder water up to 100%.

Radical polymerization can be started by initiating the decomposition of a radical polymerization initiator contained in the composition, e.g. by irradiation with UV or visible light when using a photoinitiator, e.g. Irgacure or lithium phenyl-2,4,6-trimethylbenzoyl phosphinate (LAP), or by utilizing a chemically inducible initiator composition, e.g. ammonium persulfate (APS) in combination with TEMED. Preferred radical polymerization initiators are lithium phenyl-2,4,6-trimethylbenzoylphosphinate (LAP) or Irgacure, which are activated by UV or visible light irradiation.

Any type of cross-linkable gelatin can be used for this invention including, but not limited to, e.g. (meth)acryl-amidated gelatin, thiolated and aminated gelatin, PEG-acrylate-coupled gelatin, thiobutyrolacton-coupled gelatin, preferably acrylated gelatin derivatives, which is e.g. gelatin acrylate, gelatin methacrylate or a mixture of least two of these, preferably gelatin methacrylate. The cross-linkable gelatin, e.g. gelatin acrylate and/or gelatin methacrylate, can be based on gelatin A from porcine skin, preferably Bloom factor 300, or gelatin B from bovine skin, preferably Bloom factor 225, or a mixture of these. Preferably, the cross-linkable gelatin, e.g. gelatin methacrylate, has a degree of functionalization by methacrylate groups of 50% to 100%, e.g. 50 or 100, as determined by the TNBS colorimetric assay or NMR analysis (e.g. according to Habweeb, Analytical Biochemistry 14, 3, 328-336 (1966)).

The viscosity supporting component can be any biocompatible anionic polymer which can coordinate calcium cations including, but not limited to, poly acrylic acid or carbomer. More specifically, the added carbomer is preferably is Carbopol 940, e.g. available from Acros Organics.

Generally, in the composition the acrylated gelatin derivative can be either gelatin acrylate or gelatin methacrylate

The invention is now described in greater detail by way of examples and with reference to the figures, which show in
- Fig. 1a) to f) measurement results for a composition in the cured state,
- Fig. 1g) to h) measurement results for a composition in the uncured state,
- Fig. 2 measurement results for a composition in the uncured state,
- Fig. 3a), 3b) and 3c) scanning electron microscope pictures of a cured composition,
- Fig. 4a) and b) confocal laser scanning microscopy pictures of mesenchymal stem cells (MSC) grown adherently on a 3D structure consisting of a composition of the invention and subjected to live-dead staining,
- Fig. 5a) to c) confocal laser scanning microscopy pictures of MSC grown on standard plastic cell culture vessels (TCP, Fig. 5a), a flat layer of a composition of the invention (GelMA/nHA 2D, Fig. 5b), and a 3D structure consisting of a composition of the invention (GelMA/nHA 3D, Fig. 5c),
- Fig. 6) stereomicroscopy pictures of MSC grown on standard plastic cell culture vessels (TCP, Fig. 6a and d), a flat layer of a composition of the invention (GelMA/nHA 2D, Fig. 6b and 6e), and a 3D structure consisting of a composition of the invention (GelMA/nHA 3D, Fig. 6c and 6e), and
- Fig. 7 a) to c) photographs of a printed 3D structure of a composition of the invention.

### Example 1: Bone implant produced by 3D-printing

Preferred embodiments of the composition were used for the production of implants by 3D-printing, using a temperature-controlled printing head, and/or temperature-controlled container containing the composition and connected to the printing head, also termed cartridge holder, in order to heat the composition to the suitable extrusion temperature. After the extrusion process through the printing head, the composition passed room-temperature conditions and was deposited on a platform, cooled to 5 °C. Subsequently, the composition in the form of the deposited structure, i.e. the 3D-printed composition, was irradiated with UV (365 nm) to initiate chemical cross-linking, resulting in the composition in its cured state.

These compositions comprise or consist of

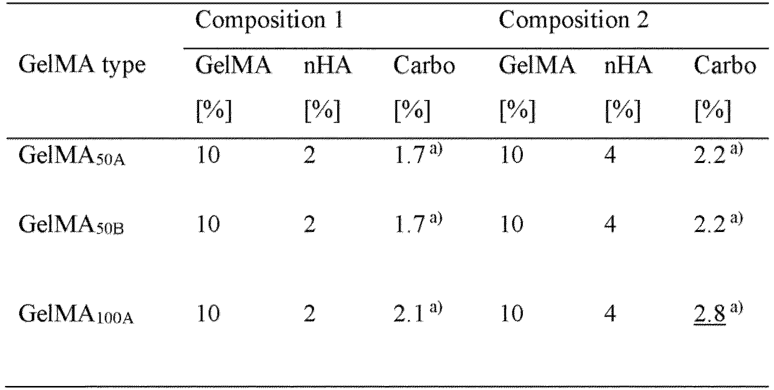

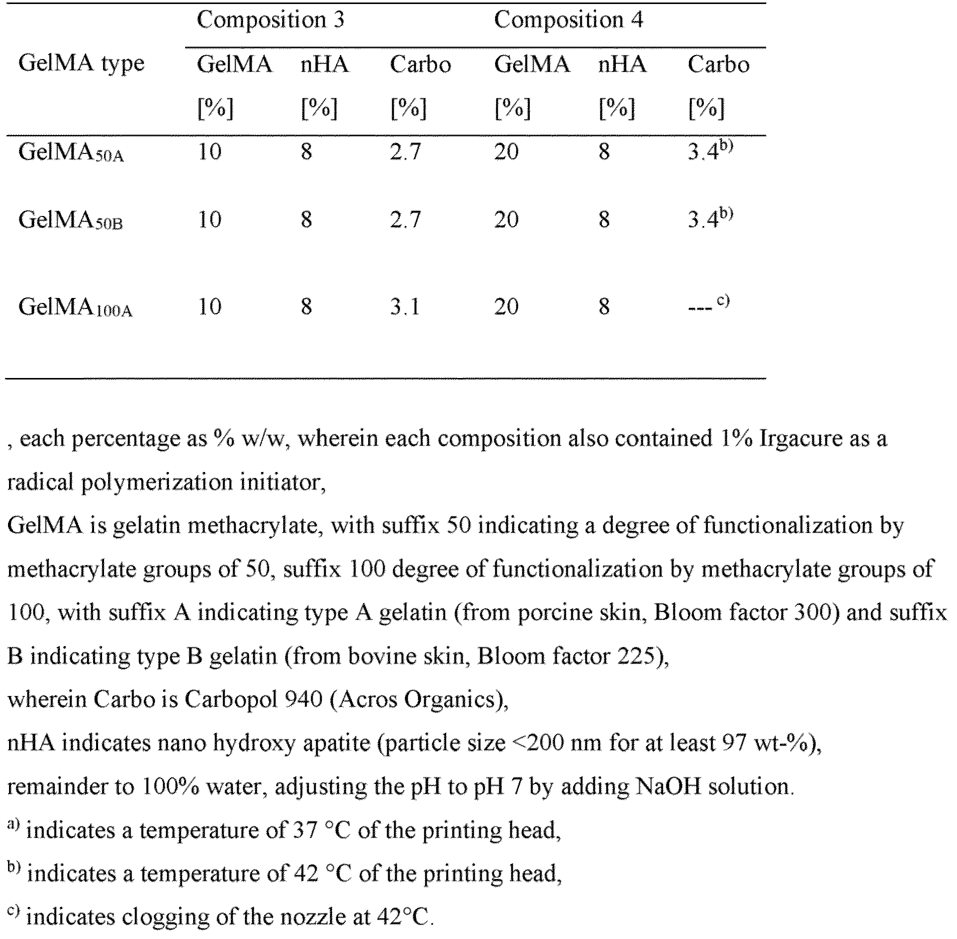

The printing head contained a heated cartridge for temperature control of the composition to the printing head temperature, and had an inner diameter of the orifice of 0.33 mm, using an applied pressure of 450 to 750 MPa for printing the composition.

Small amplitude oscillatory shear measurement using a modular MCR 302 rheometer (Antor Paar) with parallel plates, spaced by 0.5 mm, with a composition bubble-free and UV-cured after arrangement between the plates was used to determine elasticity parameters. The compositions had a shear modulus G in the desired range of 5 to 500 kPa.

All measurements were performed at 37°C, *: P < 0.05, error bars represent the SD of measurements performed on at least three samples.

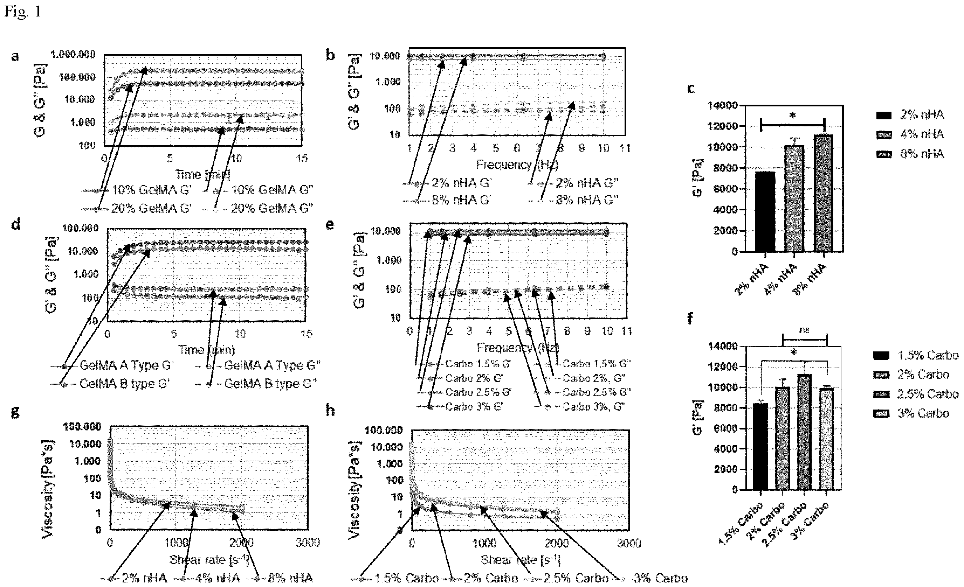

Fig. 1a) depicts data for a 15 min time sweep measurement of compositions in the cured state consisting of 10% or 20% GelMA_{100A}, 8% nHA, 3.4% carbomer, determining shear moduli in the range of 0.8 to 120 kPa.

Fig. 1b) depicts data for a frequency sweep measurement of compositions consisting of 10% w/w GelMA_{50B}, 2.7% carbomer and a concentration of nano hydroxy apatite (nHA) of 2% or 8%, showing that a higher shear modulus is obtained by a higher nHA content.

Fig. 1c) depicts data obtained from the frequency sweeps for compositions consisting of..% w/w GeIMA_{50B}, ....% carbomer and a concentration of nano hydroxy apatite (nHA) of 2%, 4% or 8%, indicating that stiffer cured compositions (higher G' values) are obtained by increasing the nHA content.

Fig. 1d) depicts data for a 15 min time sweep measurement of compositions in the cured state consisting of 10% GelMA_{50A} or 10% GelMA_{50B} , 8% nHA, 2.7% carbomer, indicating that the elastic properties can be adjusted also by the gelatine type.

Fig. 1e) depicts data obtained from frequency sweep measurements for cured compositions consisting of 10% w/w GelMA_{50B}, 4% nHA, showing that the shear modulus can be adjusted by adjusting the carbomer content.

Fig. 1f) depicts data obtained from the frequency sweeps for G' for compositions of the invention, showing that stiffness of the cured compositions generally increases with higher carbomer concentration, and that for these exemplary compositions, this effect is not significant for concentrations of more than 2%.

For compositions in the uncured state, Fig. 1g) depicts data for compositions consisting of 10% w/w GelMA_{B50}, 2.7% carbomer, with nHA at 2% or at 4% or 8%, obtained from viscosity measurements, indicating that a higher nHA concentration results in the uncured composition having a lower viscosity of the formulation.

For compositions in the uncured state, Fig. 1h) depicts data for compositions consisting of 10% w/w GelMA_{B50}, 4% nHA with carbomer at 1.5%, 2%, 2.5% or 3%, obtained from viscosity measurements in a rotating parallel plate rheometer (parallel plates, spaced by 0.5 mm) at a shear rate of 0.01 to 2000 Hz, indicating that a higher carbomer content results in an increased viscosity for the tested shear rates.

These results show that a higher content of nano hydroxyapatite increases the shear thinning property of the uncured composition, e.g. reducing its viscosity when flowing through a nozzle. It was also observed that gelation of gelatin methacrylate upon cooling is hindered even by a concentration of nano hydroxyapatite as low as 2% in the absence of carbomer. The results show that in order to adjust the composition to a desired viscosity, for instance in the case of 2% nano hydroxy apatite and 10 % of GelMA_{50A} the minimum concentration of required carbomer is at least 1.7%, preferably at least 2.1% for 10% GelMA_{100A}, for 4% nano hydroxyapatite the minimum concentration of carbomer is at least 2.2%, for 8% nano hydroxyapatite the minimum concentration of carbomer is at least 2.7%, preferably at least 3.1%. For 20% GelMA_{50A} for 8% nano hydroxy apatite the minimum concentration of carbomer is at least 3.4%.

As an alternative to forming the composition into a three-dimensional structure by 3D-printing, the composition can be injected into a mould, with solidifying the composition by cooling within the mould, followed by cross-linking, e.g., by UV-irradiation after removal of the structure from the mould or within the mould.

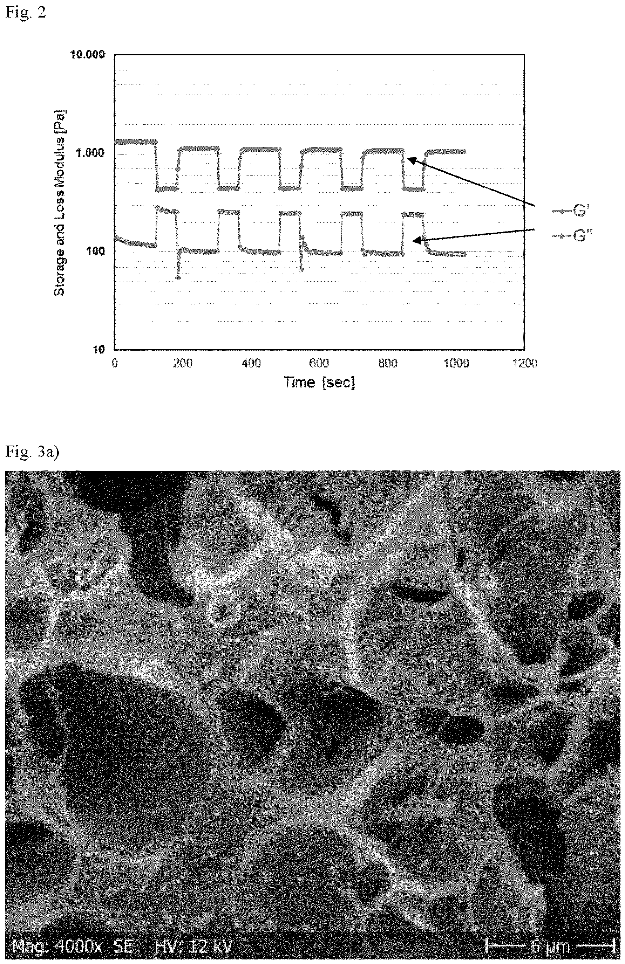

Fig. 2 depicts measurement results for compositions in the uncured state, showing self-healing properties, indicating that the compositions maintain their viscosity and elasticity properties also after treatment by an ejection through a nozzle, e.g. extrusion or a 3D-printing process. The measurement used 60 s high strain 200% deformation, followed by a 120 s recovery phase at low strain of 1%, with five repetitions of these deformation steps, each at 37°C.

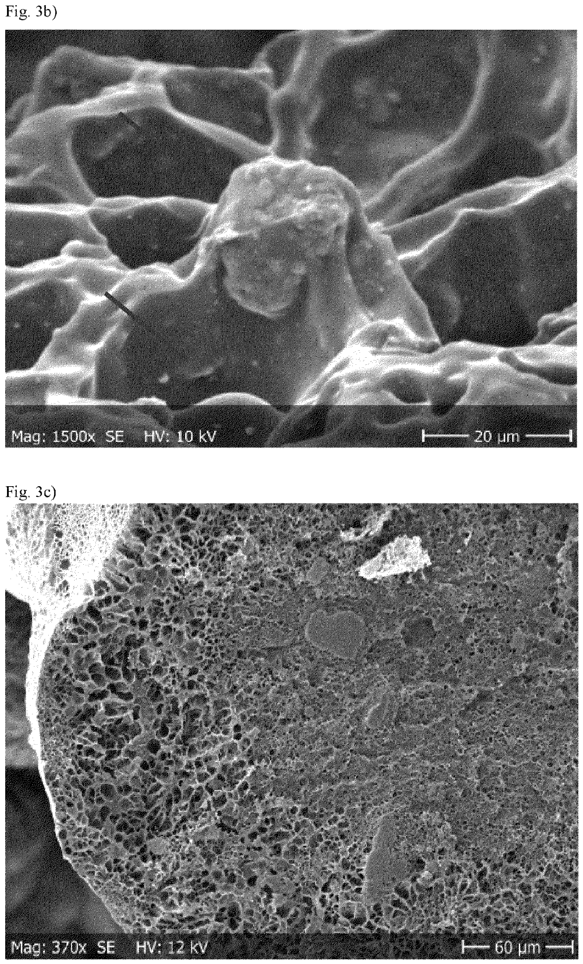

Fig. 3a), scale bar 6 µm, b), scale bar 20 µm and c), scale bar 60 µm, show the gelatin matrix in scanning electron microscope pictures of an extruded and cured composition after fixation by cooling in liquid nitrogen followed by freeze-drying. In Fig. 3b) the arrows indicate nHA particles bound to the gelatin polyacrylate matrix.

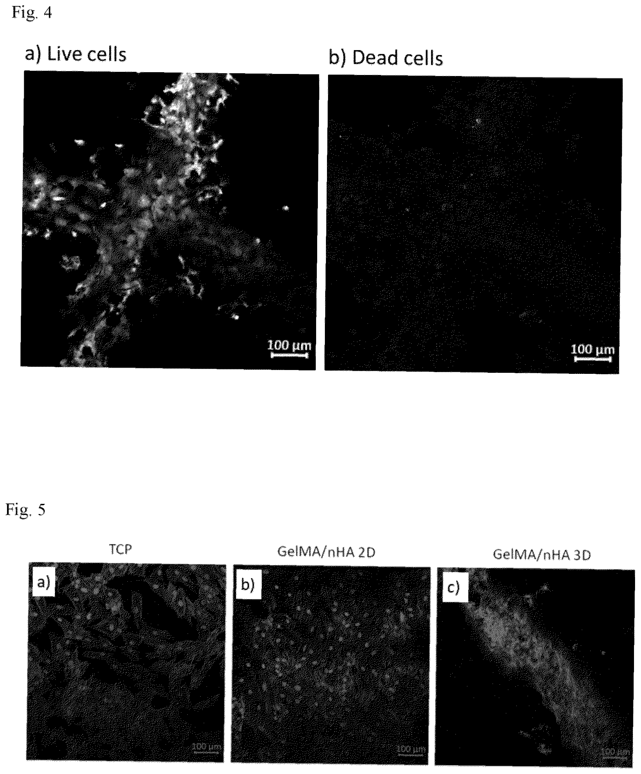

The growth of mesenchymal stem cells (MSC) and their osteogenic differentiation is shown in Fig. 4, Fig. 5 and Fig. 6, indicating that a three-dimensional structure containing recesses, e.g. an interconnected macroporous structure, consisting of a composition of the invention in its cured state supports adherent growth of cells and their differentiation to bone-producing cells.

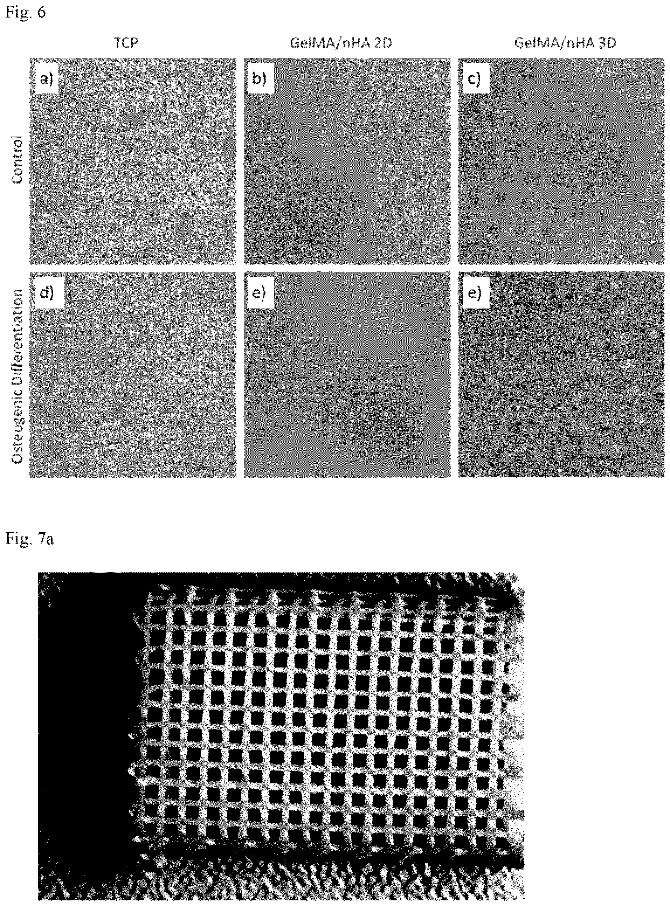

For comparison, MSC were grown in a standard plastic cell culture vessel having a flat bottom. A composition of 10% gelatin A methacrylate, 8% nanoparticular hydroxy apatite, 2.7 % Carbopol 940, and 1% Irgacure which was printed into a flat layer and UV-cured (GelMA/nHA 2D), or the same composition shaped into a grid-like structure containing interconnected macropores by 3D-printing (GelMA/nHA 3D) as shown in Fig. 5c) and in Fig. 6c) and UV-cured were arranged in a tissue culture plastic (TCP) culture 12-well plate and used for adherent growth of MSC. For the TCP controls and the GelMA/nHA 2D samples 100.000 MSC and for the GelMA/nHA 3D scaffold 500.000 MSC per well were seeded in cell culture medium (alpha MEM with stable Glutamine (P04-21250, PAN-Biotech, Aidenbach, Germany), 10 % fetal bovine serum (FBS, Sigma-Aldrich)) and incubated under static conditions at 37°C, 5% CO₂ atmosphere.

Fig. 4, scale bars 100 µm shows microscopy pictures of mesenchymal stem cells (MSC) seeded on a printed grid-like 3D structure of a composition of the invention that were subjected to live-dead staining using calcein-AM (living cells, Fig. 4a), propidium iodide (dead cells, Fig. 4b), and Hoechst33342 (nuclei). The live-dead staining shows a high number of living cells with only very few dead cells.

Fig. 5, scale bars 100 µm, shows confocal microscopy images of MSC on TCP (Fig. 5a), a flat, i.e. 2-dimensional composition (GelMA/nHA 2D, Fig. 5b), and a printed grid-like 3D structure of a composition of the invention (GelMA/nHA 3D, Fig. 5c)c) with staining for nuclei using DAPI and staining for actin by Phalloidin-Alexa488 showing adherent cell growth on all surfaces.

Fig.6, scale bars 2000 µm, shows microscopic pictures of the MSC after 21 days of cultivation on control TCP (Fig. 6a and 6d), on a flat two-dimensional (Fig. 6 b and e) and a printed grid-structure of a composition of the invention (Fig. 6 c and e), showing staining for alkaline phosphatase (dark colour), in Fig. 6a), Fig. 6b) and Fig. 6c) in cell culture medium without osteogenic differentiation factors, and in Fig. 6d), Fig. 6e) and Fig. 6f) in osteogenic differentiation medium (Human Mesenchymal Stem Cell (hMSC) Osteogenic Differentiation Medium BulletKit^{™} by Lonza, Cologne, Germany). In the medium without differentiation factors, no significant differentiation of MSC to osteogenic cells is detected, whereas in the osteogenic differentiation medium, osteogenic differentiation is seen, especially on the three-dimensional structure (Fig. 6e) of a cured composition of the invention, and less pronounced on the flat, i.e. two-dimensional, cured composition (Fig. 6e) of the invention.

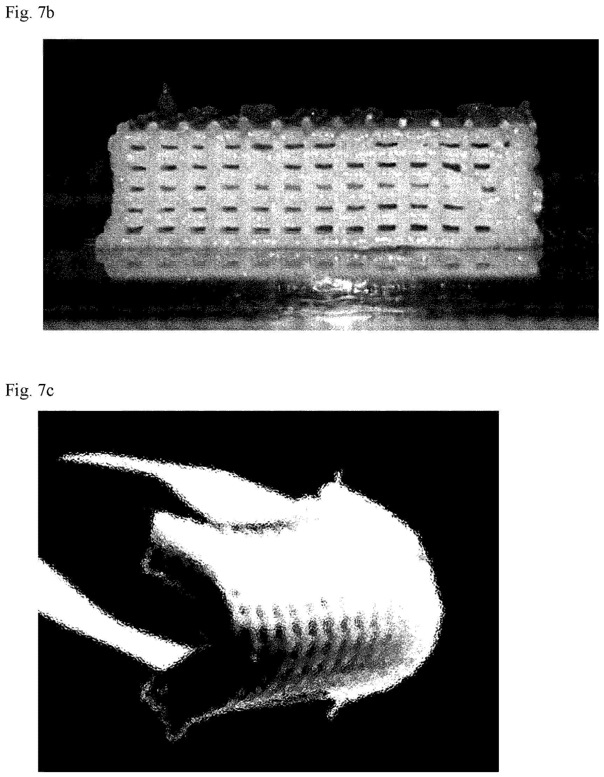

Fig. 7 shows photographs of a 3-dimensional structure with interconnected macropores, in Fig. 7a) a top view onto a grid-like structure, in Fig. 7b) a side view of the structure rotated by 90°, and in Fig. 7c) the structure bent between arms of tweezers, showing the elasticity of the structure consisting of a composition of the invention in its cured state. It was found that after bending the structure resumed its original shape.

## Claims

1. Composition comprising 5% to 30 % w/w cross-linkable gelatin, 1% to 10% w/w nanoparticular hydroxy apatite and 1% to 5% w/w Carbomer, a radical polymerization initiator, remainder water, the composition in its uncured state having a viscosity of 20 000 Pas at a shear rate of 0/s up to 1 Pas at a shear rate of 2000/s at 37°C and the composition in its cured state having a shear modulus of 5 to 500 kPa, a storage modulus G' of 10 to 300 kPa and a loss modulus G" of 0.1 to 3 kPa, each measured at 37°C by small amplitude oscillatory shear measurement.

2. Composition according to claim 1, **characterized in that** the cross-linkable gelatin is gelatin acrylate and/or gelatin methacrylate, or a mixture of these.

3. Composition according to one of the preceding claims, **characterized in that** the gelatin is based on gelatin A from porcine skin having a Bloom factor 300, or gelatin B from bovine skin having a Bloom factor 225, or a mixture of these.

4. Composition according to one of the preceding claims, **characterized in that** the viscosity of the composition in its uncured state is measured between a pair of spaced-apart parallel plates arranged with a gap of 0.5 mm, one plate of which is driven to rotate at the shear rate.

5. Composition according to one of the preceding claims, **characterized in that** the cross-linkable gelatin is gelatin acrylate or gelatin methacrylate a mixture of these is, and wherein the cross-linkable gelatin has a degree of functionalization by acrylate and/or methacrylate groups of 50 to 100 %.

6. Composition according to one of the preceding claims, **characterized in that** the nanoparticular hydroxy apatite has a particle size <200 nm for at least 97 wt-%.

7. Composition according to one of the preceding claims, **characterized in that** the composition in its uncured state has a pH in the range of 5 to 8, preferably 6 to 7.5.

8. Composition according to one of the preceding claims, **characterized in that** the pH of the composition is adjusted to pH 5 to 8, preferably 6 to 7.5.

9. Composition according to one of the preceding claims in its cured state for use in the treatment of a bone defect site by filling the bone defect site.

10. Composition for use in the treatment of a bone defect site according to claim 8 in its cured state, **characterized in that** the composition has a three-dimensional structure containing interconnected macropores.

11. Composition for use in the treatment of a bone defect site according to one of claims 8 to 9 in its cured state, **characterized in that** the treatment comprises bending the composition in its cured state from an original three-dimensional shape, inserting the composition in its bent shape into a defect site, and comprising the step of relaxation into the original three-dimensional shape until limited by the defect site.

12. Composition for use in the treatment of a bone defect site according to one of claims 1 to 9, **characterized in that** the composition in its uncured state is injected into a defect site of a bone or a defect site of cartilage and subsequently initiating radical crosslinking.

13. Process for producing a three-dimensional structure containing interconnected macropores by shaping a composition according to one of the preceding claims in its uncured state by expelling the composition through a nozzle under temperature-control to 30 to 42 °C onto a carrier or into a mould, cooling the composition, and radical cross-linking in the composition.

14. Process according to claim 13, **characterized in that** during expelling the composition through the nozzle the nozzle is moved with a spacing over the carrier.

15. Process according to one of claims 13 to 14, **characterized in that** the carrier is a cooling platform equipped with a temperature-control device.
